Europäisches Patentamt

**European Patent Office**

**Office européen des brevets**

(11) Numéro de publication: **0 002 143**

**B1**

# FASCICULE DE BREVET EUROPEEN

(21) Numéro de dépôt: **78400153.9**

(22) Date de dépôt: **27.10.78**

(51) Int. Cl.³: **C 07 D 303/04,**
**C 07 D 301/10,**
**B 01 J 23/50**

(54) **Catalyseurs à base d'argent pour la préparation d'oxyde d'éthylène**

(30) Priorité: **10.11.77 FR 7733866**

(43) Date de publication de la demande:
**30.05.79 Bulletin 79/11**

(45) Mention de la délivrance du brevet:
**21.01.81 Bulletin 81/03**

(84) Etats contractants désignés:
**BE DE FR GB NL**

(56) Documents cités:
**US - A - 4 005 049**
**US - A - 4 065 442**

(73) Titulaire: **PCUK PRODUITS CHIMIQUES UGINE**
**KUHLMANN**
**Servie Propriété Industrielle Tour Manhattan**
**F - 92087 PARIS LA DEFENSE 2 Cédex 21 (FR)**

(72) Inventeur: **Cognion, Jean-Marie**
**85 Route de Charly**
**F - 69230 St Genis Laval (FR)**
**Kervennal, Jacques**
**134 rue Docteur Locard**
**F - 69005 Lyon (FR)**

(74) Mandataire: **Monceaux, Pierre, et al**
**PRODUITS CHIMIQUES UGINE KUHLMANN**
**Service Propriété Industrielle**
**Tour Manhattan Cédex 21 F - 92087 Paris La**
**Defense (FR)**

Courier Press, Leamington Spa, England.

# 0 002 143

## Catalyseurs à base d'argent pour la préparation d'oxyde d'éthylène

La présente invention concerne des catalyseurs à base d'argent pour la préparation en phase vapeur d'oxyde d'éthylène à partir d'éthylène et d'oxygène moléculaire.

On utilise habituellement pour effectuer l'époxydation de l'éthylène des catalyseurs à base d'argent métallique déposé, avec d'éventuels promoteurs, sur des supports réfractaires. De nombreuses possibilités ont été décrites dans l'art antérieur pour améliorer l'activité et la sélectivité de ces catalyseurs. On peut, par exemple, citer le choix du support, la nature et la teneur des promoteurs et plus particulièrement la technique employée pour introduire l'argent sur le support.

Il est connu que les catalyseurs à base d'argent employés dans l'époxydation de l'éthylène, peuvent être préparés par deux techniques différentes:

a) l'enrobage du support par un composé solide de l'argent en suspension dans un liquide,

b) l'imprégnation du support par une solution d'un sel de l'argent.

Dans les deux cas les composés doivent être facilement décomposables et libérer aisément l'arget métallique sur le support. La nature des composés métalliques employés est très importante, elle peut, combinée avec la nature du support et le traitement de passage au métal, avoir une influence considérable sur l'activité et la sélectivité du catalyseur.

De nombreux composés d'argent ont été revendiqués dans l'art antérieur. On trouve par exemple décrit l'emploi, en milieu aqueux, d'un sel d'argent d'un acid carboxylique associé à une molécule organique, de préférence une amine (brevets français n° 2.117.183 et 2.253.747), l'emploi d'un sel d'argent d'hydroxy-acide comme les acides malique, isomalique, lactique, tartrique, citrique (brevets français n° 1.295.395, 2.117.183, 2.023.986) et l'emploi de divers complexes d'argent obtenus à partir de $\beta$-dicétones ou de $\beta$-céto-esters (brevet japonais n° 74.26603).

Selon l'invention les nouveaux catalyseurs sont préparés à partir de composés organométalliques "intramoléculaires" de l'argent dont les substrats organiques sont formés essentiellement d'une structure aromatique hydrocarbonée ou hétérocyclique, d'un groupement fonctionnel à caractère acide capable de former un sel d'argent et d'un atome d'azote ou d'oxygène susceptible de former une liaison de coordination avec l'atome métallique. Les groupements à caractère acide préférés sont les groupements carboxyliques et hydroxyliques. Les groupements susceptibles de former avec l'atome d'argent un complexe intramoléculaire sont choisis de préférence parmi les fonctions oxygénées cétone, ester, aldéhyde ou hydroxyle, ou les fonctions azotées amine primaire, secondaire ou tertiaire.

Comme exemples non limitatifs de substrats organiques, on peut citer les aminophénols, les acides picoloniques, les acides aminobenzoïques, les hydroxybenzaldéhydes, les acides hydroxybenzoïques, les hydroxyquinoléines et les hydroxypyridines.

De façon générale, on prépare avec de bons rendements ces complexes par réaction entre le substrat organique et un sel d'argent dont le choix influence souvent de façon importante le rendement. Le pH du milieu doit être dans tous les cas contrôlé et éventuellement ajusté par addition de soude ou d'ammoniaque.

La solubilité de ces composés organométalliques permet par une technique d'imprégnation d'obtenir des catalyseurs dans lesquels l'argent est réparti de façon uniforme. Cette bonne dispersion, favorable à l'activité et à la durée de vie des catalyseurs, est ensuite protégée par les faibles températures de décomposition de ces produits à peine supérieures aux températures de marche des catalyseurs.

La nature de ces composés organométalliques, associée à leur traitement de décomposition conduit à des tailles et à des formes de cristallites d'argent favorables à l'activité, la sélectivité et la stabilité des catalyseurs. Les observations au microscope électronique à balayage ont mis en évidence une distribution homogène et régulière de cristallites d'argent, d'aspect semisphérique, de tailles comprises entre 0,2 et 1 micron et uniformément répartis sur le support.

Ces nouveaux catalyseurs qui permettent de concilier des productivités élevées avec des sélectivités importantes en oxyde d'éthylène sont également caractérisés par une influence cinétique très faible du dioxyde de carbone.

Les catalyseurs objet de la présente invention ont été préparés par un procédé comprenant les trois étapes suivantes:

a) imprégnation d'un support réfractaire avec une solution du composé d'argent choisi. Cette imprégnation pouvant être effectuée soit par immersion du support dans la solution, soit par arrosage continu du support sous pression réduite et à une température permettant l'élimination immédiate du solvant. L'éventuelle addition des promoteurs habituels alcalins ou alcalino-terreux peut se faire à ce stade.

b) Séchage au moins partiel du support imprégné.

c) Traitement thermique du produit obtenu de manière à libérer l'argent du composé organique.

Dans cette mise au point la nature et la texture du support sont importantes. On emploie principalement des supports réfractaires comme l'alumine-$\alpha$, les silice-alumines, le carbure de silicium, la zircone. Ces supports doivent posséder une granulométrie élevée, compatible avec un emploi dans les réacteurs industriels en lit fixe. Ils se présentent sous forme d'anneaux, de pastilles, de cassons, de

# 0 002 143

sphères ou de formes extrudées, dans tous les cas de dimensions comprises entre 1/8 et 1/2 pouce (3,2 à 13 mm) et de propriétés mécaniques satisfaisantes, ne permettant en aucun cas la formation de poussières au cours de la préparation du catalyseur, de ses manipulations ou de son fonctionnement. Les surfaces spécifiques de ces produits — mesurées par la technique dite de BRUNAUER, EMMETT et TELLER, décrite dans le Journal of American Chemical Society, vol. 60, page 309, 1938 — sont inférieures à 10 m2/g mais l'on préfère des surfaces encore plus faibles, inférieures à 2 m2/g et plus particulièrement comprises entre 0,1 et 1 m2/g.

Les porosités apparentes principalement formées de pores de rayons supérieurs à 0,25 microns, doivent être au minimum de 40 à 50% en volume.

On a principalement utilisé pour ces essais trois supports commercialisés par la firme NORTON, possédant les caractéristiques du tableau n° 1.

TABLEAU n° 1

Vp : volume de porosité en cm3/g

$R_M$ : rayon moyen des pores en $\mu$

| SUPPORT | LA 956 | SA 5151 | SA 5205 |
|---|---|---|---|
| forme granulométrie | extrudée 1/4 × 1/4 pouce (6,4 × 6,4 mm) | extrudée 1/4 × 1/4 pouce (6,4 × 6,4 mm) | sphères 1/4 pouce (6,4 mm) |
| $Al_2O_3$ % | 99,3 | 99,3 | 86,1 |
| $SiO_2$ % | 0,3 | 0,4 | 11,8 |
| CaO % | 0,1 | 0,1 | 0,4 |
| $Na_2O$ % | 0,1 | 0,1 | 0,4 |
| $K_2O$ % | 0,02 | — | 0,6 |
| Surface spécifique BET (m2/g) | 0,20 | 0,20 | 0,30 |
| POROSITE : | | | |
| Vp total | 0,170 | 0,270 | 0,100 |
| Vp (R = 0,25 à 5 $\mu$) | 0,150 | 0,240 | 0 |
| | (88 %) | (88 %) | 0 |
| Vp (R = 5 à 100 $\mu$) | 0,020 | 0,030 | 0,100 |
| | (12 %) | (12 %) | (100 %) |
| $R_M$ des pores | 1 | 2 | 55 |

L'imprégnation des supports a été effectuée dans un évaporateur rotatif. La solution du composé métallique employé est soit ajoutée en une seule fois sur le support et le solvant chassé sous pression réduite, soit introduite lentement sur le support de façon à ce que l'évaporation du solvant soit immédiate. La teneur en composé métallique de la solution est fonction de sa solubilité et la teneur en argent du catalyseur est déterminée par la quantité de solution employée. En général le catalyseur contient de 5 à 25% d'argent, mais préférentiellement, entre 8 et 20%. Les produits imprégnés sont

3

# 0 002 143

alors transférés dans un réacteur tubulaire, pour y subir un traitement de décomposition thermique du composé organométallique qui libère l'argent. Ce traitement se fait de préférence sous balayage d'azote, d'oxygène, d'hydrogène ou de mélanges oxygène-azote ou hydrogène-azote.

Nous avons également vérifié, en accord avec l'art antérieur, que certains composés comme les dérivés halogénés des hydrocarbures augmentaient la sélectivité de nos catalyseurs, lorsqu'ils étaient ajoutés en faibles quantités aux réactifs. Nous avons particulièrement utilisé le dichloro-1,2 éthane avec une concentration maximale de 1 ppm par rapport au volume gazeux total.

## RESULTATS

Pour chaque essai nous avons calculé:

— le taux de transformation globale de l'éthylène (T.T.G.):

$$\text{T.T.G.} = \frac{\text{Nbre de moles d'éthylène transformées}}{\text{Nbre de moles d'éthylène introduites}} \times 100$$

— la sélectivité de la transformation en oxyde d'éthylène (S.O.E.)

$$\text{S.O.E.} = \frac{\text{Nbre de moles d'oxyde d'éthylène formées}}{\text{Nbre de moles d'éthylène transformées}} \times 100$$

### Exemple 1

A une solution de 10,6 g, soit 0,08 mole, d'acide anthranilique dans 140 ml d'éthanol à 50°C, on ajoute une solution aqueuse également à 50°C de 12,9 g, soit 0,08 mole, d'acétate d'argent dans 750 ml d'eau. Le complexe qui précipite immédiatement est, après refroidissement, filtré, lavé à l'eau et séché. On recueille 18 g d'anthranilate d'argent, ou orthoaminobenzoate d'argent, correspondant à un rendement molaire de 95%. La teneur en argent correspond à la théorie: 44,2%.

On place dans un ballon à solides, monté sur un évaporateur rotatif, 42,5 g d'un support 5151 fabriqué par la société NORTON et dont les caractéristiques sont rassemblées dans le tableau 1. On porte la température du bain d'huile de l'évaporateur à 120°C, et on laisse dégazer le support pendant une heure sous une pression partielle de 100 mm de mercure. Dans les mêmes conditions de température et de pression, on introduit ensuite, goutte à goutte, sur le support agité, 300 ml d'une solution pyridinique de 18 g du complexe anthranilate d'argent précédemment préparé. Dans ces conditions, l'évaporation du solvant est instantanée. Après introduction de la totalité de la solution, le support, imprégné et séché est transféré dans un réacteur tubulaire pour décomposer le complexe dans un courant d'azote auquel on ajoute 1,5% d'hydrogène. Afin de contrôler thermiquement la réaction, ce traitement est effectué avec une montée de température de 20°C/heure jusqu'à un palier de 18 heures à 280°C. L'analyse indique que le catalyseur obtenu contient 9% en poids d'argent. On charge un réacteur de laboratoire travaillant à la pression atmosphérique avec 30 ml du catalyseur.

La réacteur est constitué par un tube en acier inoxydable de 600 mm de long et de 16 mm de diamètre intérieur. Les réactifs sont admis par le bas et sont préchauffés sur un lit d'anneaux de porcelaine de 200 mm de haut, qui supporte également la charge de catalyseur. Le chauffage de l'ensemble est assuré par une circulation d'huile dans une double enveloppe. Les gaz, entrant et sortant du réacteur, sont analysés en ligne à l'aide d'un chromatographe à double réactin: un détecteur à ionisation de flamme pour l'oxyde d'éthylène, le méthane, le formol, le propylène, le propane, le méthanol et l'acétaldéhyde et un détecteur de conductibilité thermique pour l'oxygène-azote, le dioxyde de carbone, l'éthylène et l'eau. Deux colonnes de diamètre 1/8 pouce et de longueur 2,5 mètres, montées en série, sont remplies l'une de chromosorb 101, l'autre de porapak Q.

Après un traitement d'activation de 12 heures, consistant à faire passer sur le catalyseur un mélange air-éthylène 50%—50% à la température de 218°C, on introduit un courant gazeux de 14 litres/heure, constitué de 14% d'éthylène, 4,5% d'oxygène, 81,5% d'azote et de 400 parties par billion de dichloro-1,2 éthane. Après 66 heures de marche on obtient les résultats du tableau n° 2.

4

TABLEAU n° 2

| T°C du catalyseur | % T.T.G. | % S.O.E. |
|---|---|---|
| 203 | 5 | 73 |
| 225 | 10 | 67 |

## Exemple 2

Conformément à la synthès décrite dans l'article de R.G.R. BACON et W.J.W. HANNA dans le Journal of the Chemical Society, 1965, page 4962, le picolinate d'argent, ou sel de l'acide pyridine carboxylique-2 peut être préparé en faisant réagir, mole à mole, l'acide picolinique et le nitrate d'argent en solution aqueuse. Le complexe, partiellement soluble dans le milieu, précipite avec un rendement d'environ 75%.

On prépare selon la technique décrite ci-dessus, une solution de picolinate d'argent dans la pyridine en faisant réagir en milieu aqueux 11,8 g, soit 0,07 mole, de nitrate d'argent, et 8,55 g soit 0,07 mole d'acide picolinique. Le précipité filtré, lavé à l'eau et séché est dissous sur filtre dans la pyridine.

On charge dans un ballon à solides, monté sur un évaporateur rotatif 42,5 g de support LA 956 fabriqué par la société NORTON et dont les caractéristiques sont rassemblées dans le tableau 1. On y ajoute la solution préparée précédemment et on dégaze l'ensemble à température ambiante, sous une pression partielle de 100 mm de mercure pendant une heure. On distille ensuite le solvant sous une pression partielle de 15 à 20 mm de mercure avec une température de bain d'huile de l'évaporateur de 50°C. Le support imprégné est séché puis chargé dans un réacteur tubulaire pour décomposition du complexe métallique en deux temps: tout d'abord chauffage sous azote, 8 heures à 270°C et ensuite montée progressive de 200 à 270°C sous balayage d'un mélange azote-oxygène à 10% d'oxygène. L'analyse indique une teneur en argent du catalyseur de 6% en poids.

On charge 30 ml de ce catalyseur dans un réacteur de laboratoire fonctionnant sous pression constitué essentiellement par un tube en acier inoxydable, de 355 mm de long et de 16 mm de diamètre intérieur chauffé par un bain de nitrates fondus. Les réactifs admis par le bas du réacteur sont préchauffés sur un remplissage en porcelaine de 42 mm de hauteur. Le dispositif analytique est identique à celui décrit dans l'exemple 1. On fait tout d'abord subir au catalyseur un prétraitement d'activation par un mélange éthylène-air 50%—50%, à la pression atmosphérique, pendant 30 heures à une température croissante de 150 à 190°C. On admet, ensuite, sous une pression de 20 bars, les réactifs constitués par un mélange de 13% d'éthylène, 5% d'oxygène, 82% d'azote et 35 parties par billion de dichloro-1-2 éthane, à un débit de 9.000 litres normaux par heure par litre de catalyseur. Après 30 heures de marche on obtient à 193°C un taux de transformation de l'éthylène de 5% avec une sélectivité en oxyde d'éthylène de 76%.

## Exemple 3

On charge dans un ballon à solides, monté sur un évaporateur rotatif 42,5 g de support LA 956 fabriqué par la société NORTON et dont les caractéristiques sont rassemblées dans le tableau n° 1. On y ajoute une solution de 17,5 g de picolinate d'argent préparé comme indiqué à l'exemple 2 dans 420 g de pyridine et on dégaze l'ensemble, à température ambiante, sous une pression partielle de 100 mm de mercure pendant une heure. On distille ensuite le solvant sous une pression partielle de 290 mm de mercure avec une température de bain d'huile de l'évaporateur de 115°C. Comme dans l'exemple 2, la décomposition du complexe métallique est effectuée en deux étapes: tout d'abord chauffage sous azote à 20°C/Heure jusqu'à un palier de 3 heures à 270°C et ensuite traitement de 38 heures par un mélange azote-oxygène à 10—12% d'oxygène, à 270°C. L'analyse indique une teneur en argent du catalyseur de 11,8% en poids.

On charge 30 ml de ce catalyseur dans le réacteur décrit dans l'exemple 2. On introduit, dans un premier temps, un courant gazeux, contenant 13% d'éthylène, 5% d'oxygène, 82% d'azote et 35 ppb (parties par billion) de dichloro-1,2 éthane à un débit horaire spécifique de 9.000 litres normaux par litre de catalyseur. Après 30 heures de marche on obtient les résultats du tableau 3. On remplace ensuite 11% d'azote par 11% de dioxyde de carbone et on obtient après 10 heures de marche les résultats du tableau n° 3.

TABLEAU n° 3

| T°C du catalyseur | % $CO_2$ dans les réactifs | % T.T.G. | % S.O.E. |
|---|---|---|---|
| 214 | 0 | 5 | 75 |
| 220 | 11 | 5 | 73 |

## Exemple 4

Pour préparer le complexe de l'argent avec le salicylaldéhyde ou orthohydroxybenzaldéhyde, on ajoute à une solution de 7,2 g, soit 0,042 mole, de nitrate d'argent dans 40 ml d'eau, une solution de 5,2 g, soit 0,042 mole, de salicylaldéhyde dans 30 ml d'éthanol et ensuite, goutte à goutte, une solution de 1,68 g de soude dans 70 ml d'eau. A pH = 7,7, il se forme un précipité jaune-vert qu'on filtre. On reprend le filtrat dans lequel on continue à ajouter, goutte à goutte, la solution de soude jusqu'à ce que le pH atteigne à nouveau 7,7 et qu'apparaisse un nouveau précipité. On recueille celui-ci avec le précédent et on recommence l'opération jusqu'à épuisement de la soude. L'addition de celle-ci doit être effectuée avec précautions de façon à éviter le passage brutal à un pH trop basique entraînant la précipitation d'oxyde d'argent. Après plusieurs lavages à l'eau désoxygénée et à l'éthanol du précipité, on recueille par séchage 8,2 g d'un produit vert contenant 46,9% en poids d'argent, pour une valeur théorique de 47,1%. Le rendement molaire est de 85%. Le produit doit être conservé à l'abri de l'air et de la lumière, car il subit une lente décomposition.

Suivant le mode opératoire décrit dans l'exemple 1, on prépare un catalyseur à partir d'une solution de 8,2 g du complexe d'Ag du salicylaldéhyde ainsi obtenu dans 150 ml de pyridine et de 36 g de support 5205 fabriqué par la société NORTON et dont les caractéristiques sont rassemblées dans le tableau n° 1. L'analyse indique que la teneur en Ag est de 6,8%.

On place 30 ml de ce catalyseur dans le réacteur de l'exemple 1 et on fait passer pendant 45 heures un mélange air-éthylène 50%—50% à une température entre 182 et 227°C. On introduit ensuite dans le réacteur un courant gazeux de 14 l/h constitué d'un mélange de 14,8% d'éthylène, 4,6% d'oxygène, 80,6% d'azote et 100 parties par billion de dichloro-1,2 éthane. Après 15 heures de marche, on obtient les résultats du tableau 4.

TABLEAU n° 4

| T°C du catalyseur | % T.T.G. | % S.O.E. |
|---|---|---|
| 222 | 5 | 72 |
| 246 | 10 | 69 |

## Exemple 5

On prépare le sel d'argent de l'acide salicylique en ajoutant une solution de 7,9 g, soit 0,046 mole de nitrate d'argent dans 15 ml d'eau à une solution de 6,3 g, soit 0,046 mole, d'acide salicylique dans 100 ml d'éthanol. On coule ensuite lentement dans le mélange une solution d'ammoniaque 5N jusqu'à pH 7. Le précipité blanc qui se forme immédiatement est lavé à l'eau, filtré et séché. On recueille 10,6 g de salicylate d'argent correspondant à un rendement molaire de 94%. La teneur en argent correspond à la théorie = 44%.

Suivant le mode opératoire décrit dans les exemples 1 et 4, on prépare un catalyseur à partir d'une solution pyridinique de 10,6 du salicylate d'argent préparé ci-dessus et 42,5 g de support 5151 fabriqué par la société NORTON et dont les caractéristiques sont rassemblées dans le tableau n° 1. La teneur en Ag du catalyseur obtenu est de 8,7%.

On place 30 ml de ce catalyseur dans le réacteur décrit dans l'exemple 1 et on fait passer pendant 47 heures un mélange air-éthylène 50%—50% à 174°C. On introduit ensuite 14 litres/heure de gaz contenant 13% d'éthylène, 4,7% d'oxygène, 82,3% d'azote et 100 ppb de dichloro-1,2 éthane. Après 10 heures de marche, on obtient les résultats du tableau n° 5.

6

TABLEAU n° 5

| T°C du catalyseur | % T.T.G. | % S.O.E. |
|---|---|---|
| 161 | 5 | 68 |
| 179 | 10 | 66 |

## Revendications

1. Catalyseurs de synthèse de l'oxyde d'éthylène par oxydation en phase vapeur de l'éthylène par l'oxygène ou par un mélange gazeux en contenant, préparés à partir de composés organométalliques de l'argent déposés par imprégnation sur un support réfractaire, catactérisés en ce que les substrats organiques de ces composés sont essentiellement formés d'une structure à caractère aromatique hydrocarbonée ou hétérocyclique, d'un groupement fonctionnel à caractère acide formant un sel d'argent et d'un atome d'azote ou d'oxygène susceptible de former une liaison de coordination avec l'atome métallique.

2. Catalyseurs selon la revendication 1 préparés à partir de composés organométalliques de l'argent, caractérisés en ce que le substrat organique est constitué d'un hydrocarbure aromatique substitué par un groupement fonctionnel carboxylique et par un groupement fonctionnel azoté ou oxygéné susceptible de former avec l'atome métallique un complexe intramoléculaire par coordination.

3. Catalyseurs selon la revendication 1 préparés à partir de composés organométalliques de l'argent, caractérisés en ce que le substrat organique est constitué d'un hydrocarbure aromatique substitué par un groupement fonctionnel hydroxylique et par un groupement fonctionnel azoté ou oxygèné susceptible de former avec l'atome métallique un complexe intramoléculaire par coordination.

4. Catalyseurs selon l'une des revendications 2 et 3, caractérisés en ce que le groupement susceptible de former avec l'atome métallique un complexe intramoléculaire est une fonction cétone, ester, aldéhyde ou hydroxyle.

5. Catalyseurs selon l'une des revendications 2 et 3, caractérisés en ce que le groupement susceptible de former avec l'atome métallique un complexe intramoléculaire est une fonction amine primaire, secondaire ou tertiaire.

6. Catalyseurs selon la revendication 1, préparés à partir de composés organométalliques de l'argent, caractérisés en ce que le substrat organique est constitué d'un hétérocycle à caractère aromatique, comprenant dans sa structure un atome d'oxygène et/ou d'azote, et substitué par un groupement fonctionnel acide, hydroxylique ou carboxylique.

7. Catalyseurs selon la revendication 4, caractérisés en ce qu'ils sont préparés à partir du sel d'argent de l'acide salicylique.

8. Catalyseurs selon la revendication 5, caractérisés en ce qu'ils sont préparés à partir du sel d'argent de l'acide anthranilique.

9. Catalyseurs selon la revendication 4, caractérisés en ce qu'ils sont préparés à partir du sel d'argent du salicylaldéhyde.

10. Catalyseurs selon la revendication 6, caractérisés en ce qu'ils sont préparés à partir du sel d'argent de l'acide picolinique.

11. Catalyseurs selon l'une des revendications 1 à 10, caractérisés par l'utilisation de supports réfractaires alumineux et silico-alumineux de surface spécifique inférieure à 2 m2/g et de porosité apparente d'au moins 40% en volume, principalement formée de pores de rayons supérieurs à 0,25 microns.

12. Catalyseurs selon l'une des revendications 1 à 11, caractérisés en ce qu'ils contiennent de 5 à 25% d'argent.

13. Procédé de préparation d'oxyde d'éthylène par oxydation de l'éthylène en phase vapeur par l'oxygène, ou par un mélange gazeux en contenant, caractérisé par l'emploi comme catalyseur d'un catalyseur selon l'une des revendications 1 à 12.

## Patentansprüche

1. Katalysatoren für die Synthese von Äthylenoxid durch Dampfphasenoxidation von Äthylen mit Sauerstoff oder Sauerstoff enthaltenden Gasen, hergestellt aus Organometallverbindungen des Silbers, die durch Tränken auf einen feuerfesten Träger aufgebracht wurden, dadurch gekennzeichnet, daß die organischen Substrate dieser Verbindungen im wesentlichen aus einer Struktur mit aromatischem Kohlenwasserstoff —— oder heterocyclischem Charakter, einer funktionellen sauren Gruppe, die ein Silbersalz bildet und einem Stickstoff- oder Sauerstoffatom gebildet werden, das eine Coordinationsverbindung mit dem Metallatom eingehen kann.

2. Katalysatoren nach Anspruch 1, hergestellt aus Organometallverbindungen des Silbers, dadurch gekennzeichnet, daß das organische Substrat aus einem aromatischen Kohlenwasserstoff besteht der durch eine Gruppe mit Carboxylfunktion und eine funktionelle Stickstoff oder Sauerstoff enthaltende Gruppe substituiert ist, die mit dem Metallatom durch Coordination einen intramolekularen Komplex bilden kann.

3. Katalysatoren nach Anspruch 1, hergestellt aus Organometallverbindungen des Silbers, dadurch gekennzeichnet, daß das organische Substrat aus einem aromatischen Kohlenwasserstoff besteht, der durch eine Gruppe mit Hydroxylfunktion und eine Stickstoff oder Sauerstoff enthaltende funktionelle Gruppe substituiert ist, die mit dem Metallatom durch Coordination einen intramolekularen Komplex bilden kann.

4. Katalysatoren nach einem der Ansprüche 2 oder 3, dadurch gekennzeichnet, daß die Gruppe, die mit dem Metallatom einen intramolekularen Komplex bilden kann, ein Keton, Ester, Aldehyd oder Hydroxid ist.

5. Katalysatoren nach einem der Ansprüche 2 oder 3, dadurch gekennzeichnet, daß die Gruppe, die mit dem Metallatom einen intramolekularen Komplex bilden kann, ein primäres, sekundäres oder tertiäres Amin ist.

6. Katalysatoren nach Anspruch 1, hergestellt aus einer Organometallverbindung des Silbers, dadurch gekennzeichnet, daß das organische Substrat aus einem Heterozyklus mit aromatischen Charakter besteht und in seiner Struktur ein Sauerstoff- und/oder Stickstoffatom hat und durch eine saure, hydroxylische oder carboxylische funktionnelle Gruppe substituiert ist.

7. Katalysatoren nach Anspruch 4, dadurch gekennzeichnet, daß sie aus einem Silbersalz der Salicylsäure hergestellt wurden.

8. Katalysatoren nach Anspruch 5, dadurch gekennzeichnet, daß sie aus einem Silbersalz der Anthranilsäure hergestellt wurden.

9. Katalysatoren nach Anspruch 4, dadurch gekennzeichnet, daß sie aus einem Silbersalz des Salicylaldehyds hergestellt wurden.

10. Katalysatoren nach Anspruch 6, dadurch gekennzeichnet, daß sie aus einem Silbersalz der Picolinsäure hergestellt wurden.

11. Katalysatoren nach einem der Ansprüche 1 bis 10, gekennzeichnet durch die Verwendung von feuerfesten Alumino- und Silicoaluminoträgern mit einer spezifischen Oberfläche unter 2 m²/g und einer scheinbaren Porosität von wenigstens 40 Vol.%, die vorwiegend aus Poren mit einem Radius über 0,25 $\mu$m gebildet werden.

12. Katalysatoren nach einem der Ansprüche 1 bis 11, dadurch gekennzeichnet, daß sie 5 bis 25% Silber enthalten.

13. Verfahren zur Herstellung von Äthylenoxid durch Dampfphasenoxydation von Äthylen mit Sauerstoff oder einem sauerstoffhaltigen Gasgemisch, gekennzeichnet durch die Verwendung eines Katalysators nach einem der Ansprüche 1 bis 12.

## Claims

1. Catalysts for the synthesis of ethylene oxide by oxidation, in the vapour phase, of ethylene by oxygen or a gaseous mixture containing oxygen, said catalysts being prepared from organometallic silver compounds deposited by impregnation on a refractory support, characterised in that the organic substrates of these compounds are essentially formed from a structure of a heterocyclic or aromatic hydrocarbon type, a functional group of acidic nature forming a silver salt and a nitrogen or oxygen atom capable of forming a coordinating bond with the metal atom.

2. Catalysts according to claim 1, prepared from organometallic silver compounds, characterised in that the organic substrate consists of an aromatic hydrocarbon substituted by a functional carboxyl group and by a functional nitrogen or oxygen group capable of forming an intramolecular complex with the metal atom by coordination.

3. Catalysts according to claim 1, prepared from organometallic silver compounds, characterised in that the organic substrate consists of an aromatic hydrocarbon substituted by a functional hydroxyl group and by a functional nitrogen or oxygen group capable of forming an intramolecular complex with the metal atom by coordination.

4. Catalysts according to one of claims 2 and 3, characterised in that the group capable of forming an intramolecular complex with the metal atom is a ketone, ester, aldehyde or hydroxide function.

5. Catalysts according to one of claims 2 and 3, characterised in that the group capable of forming an intramolecular complex with the metal atom is a primary, secondary or tertiary amine function.

6. Catalysts according to claim 1, prepared from organometallic silver compounds, characterised in that the organic substrate consists of a heterocycle of aromatic nature, comprising in its structure an oxygen and/or nitrogen atom, and substituted by a functional acid, hydroxyl or carboxyl group.

7. Catalysts according to claim 4, characterised in that they are prepared from the silver salt of salicylic acid.

8. Catalysts according to claim 5, characterised in that they are prepared from the silver salt of anthranilic acid.

9. Catalysts according to claim 4, characterised in that they are prepared from the silver salt of salicylaldehyde.

10. Catalysts according to claim 6, characterised in that they are prepared from the silver salt of picolinic acid.

11. Catalysts according to one of claims 1 to 10, characterised by the use of aluminous and silico-aluminous refractory supports with a specific surface area of less than 2 $m^2/g$ and with an apparent porosity of at least 40% by volume, principally formed by pores with radii of more than 0.25 microns.

12. Catalysts according to one of claims 1 to 11, characterised in that they contain from 5 to 25% silver.

13. Method of preparing ethylene oxide by oxidising ethylene in the vapour phase using oxygen or a gaseous mixture containing oxygen, characterised by the use, as catalyst, of a catalyst according to one of claims 1 to 12.